# FASCICULE DE BREVET EUROPEEN

(11) **EP 1 266 645 B1**
(45) Date de publication et mention de la délivrance du brevet: **11.01.2006**
(21) Numéro de dépôt: 02291386.7
(22) Date de dépôt: 05.06.2002
(51) Int. Cl.: A61J 1/00, A61M 1/02

(54) **Système à poches comprenant au moins une poche pourvue de moyens d'association temporaire d'un filtre**
Beutelsystem mit mindestens einem Beutel mit Mitteln zum temporären Halten eines Filters
Bag system comprising at least one bag with means for temporarily associating a filter

(30) Priorité: 12.06.2001 FR 0107679
(43) Date de publication de la demande: 18.12.2002
(73) Titulaire: MACO PHARMA, 59420 Mouvaux (FR)
(72) Inventeur: Goudaliez, Francis, 59155 Faches-Thumesnil (FR); Verpoort, Thierry, 59420 Mouvaux (FR); Ribeiro, Gus, Narraweena, NSW (AU)
(74) Mandataire: Geismar, Thierry

(56) Documents cités:
- EP-A- 0 426 475
- EP-A- 0 976 413
- DE-A- 1 966 731
- DE-A- 3 906 418
- US-A- 4 735 613
- US-A- 5 836 934

## Description

L'invention concerne une poche souple destinée à recevoir un fluide biologique et un système à poches comprenant une telle poche.

Une telle poche est destinée notamment à recevoir du sang ou un composant du sang.

On connaît, notamment du document US-4 767 541, l'intégration d'une telle poche dans un système clos en vue de séparer le sang en ses composants.

A cet effet, le système comprend typiquement des moyens de prélèvement du sang, une poche primaire destinée à recueillir le sang, et, reliés à celle-ci par l'intermédiaire de tubulures, au moins un filtre et au moins une poche secondaire destinée à recevoir le filtrat.

La mise en oeuvre d'un tel système fait intervenir successivement une étape de centrifugation pour séparer le sang en ses différents composants, à savoir le plasma, les globules blancs ou leucocytes, et les globules rouges, et une étape de filtration d'au moins un composant du sang, afin d'éliminer les leucocytes. Il est en effet connu que les leucocytes sont indésirables dans les composants sanguins à transfuser.

Lors de l'étape de centrifugation, il est nécessaire d'introduire dans le bol de centrifugation non seulement les diverses poches souples, mais également le filtre.

Or il s'avère que, lors de cette étape, le filtre risque d'être endommagé, compte-tenu de l'accélération et de la vitesse de centrifugation : il peut en effet se plier, créant ainsi des passages préférentiels pour le sang, ou bien se casser, ce qui rend par conséquent la filtration inopérante.

En outre, et notamment lorsque le filtre a une enveloppe rigide, la centrifugation de celui-ci conjointement avec les poches souples et les tubulures peut induire, par frottement, une détérioration de celles-ci. Dans certains cas, cette détérioration peut provoquer un percement d'une poche et/ou d'une tubulure, ce qui rend le système inutilisable pour la séparation du sang en ses composants.

Pour remédier à ces problèmes, différents systèmes ont déjà été proposés. On peut les classer en deux catégories :
- les systèmes nécessitant un agencement particulier du bol de centrifugation, par exemple en insérant dans le bol un support rigide destiné à accueillir le filtre pendant l'étape de centrifugation, et donc séparer le filtre des autres éléments du système ;
- les systèmes faisant appel à des moyens externes aux bols de centrifugation, à savoir, par exemple, des moyens d'association du filtre sur le bol.

Les systèmes de la première catégorie présentent notamment l'inconvénient de nécessiter un type de support particulier pour chaque bol de centrifugation. En outre, la présence du support rigide à l'intérieur du bol limite la place disponible pour le système.

Il est connu de prévoir, pour les systèmes de la deuxième catégorie, soit des moyens d'association intégrés au filtre ou extérieurs à celui-ci. Dans le premier cas, la réalisation du filtre est rendue plus compliquée. Dans le deuxième cas, on utilise notamment des rubans adhésifs qui assurent une association peu fiable.

Par ailleurs, il est connu du document US-5 836 934 d'associer un étui sur une poche afin de loger un filtre durant la centrifugation. Cette solution présente notamment l'inconvénient de ne pas être simple de mise en oeuvre en ce qu'elle nécessite la réalisation d'un étui spécifique qui doit être associé à la poche lors d'une étape spécifique. En outre, l'association est réalisée sur une face de la poche qui, lors du remplissage de la poche se déforme. Ainsi une perte de fiabilité de l'association peut être occasionnée, notamment lors des efforts importants qui sont induits par la centrifugation.

Pour pallier ces inconvénients, l'invention propose notamment des moyens d'association temporaire du filtre sur l'une des poches du système, qui permettent une association fiable et aisée de celui-ci sans augmenter sensiblement la difficulté de réalisation et le coût de ladite poche. Ainsi, lors de l'étape de centrifugation, la poche et le filtre forment temporairement un ensemble compact qui permet d'assurer la protection des différents éléments du système, en évitant notamment les frottements entre eux, ainsi que le déplacement du filtre lors de la centrifugation.

En outre, cet ensemble est de manipulation aisée, puisqu'il peut être placé dans le bol de centrifugation, puis retiré une fois la centrifugation terminée, de façon simple et rapide, et sans risquer de mélanger les composants centrifugés.

De plus, le système peut être mis en oeuvre avec les types de bols disponibles commercialement.

Selon un premier aspect, l'invention concerne un système à poches, tel que revendiqué dans la revendication 1, comprenant une poche souple destinée à recevoir un fluide biologique en vue de sa centrifugation, et un filtre connecté à ladite poche par l'intermédiaire d'une tubulure, ladite poche souple comprenant une enveloppe extérieure formée de deux feuilles de plastique souple assemblées sur leur périphérie de sorte à définir un volume intérieur pour ledit fluide, ladite enveloppe extérieure étant munie d'au moins un orifice d'entrée et/ou de sortie du fluide, ladite poche comprenant en outre des moyens d'association temporaire du filtre avec ladite poche, lesdits moyens étant disposés sur l'enveloppe extérieure, ledit système étant tel que les moyens d'association temporaire sont assemblés sur la poche sur une partie de la périphérie de l'enveloppe et sont agencés pour permettre l'insertion dudit élément entre lesdits moyens et ladite enveloppe de sorte à assurer l'association temporaire lors de la centrifugation de la poche.

Selon un mode de réalisation, les moyens d'association temporaire sont formés d'une bande de matière plastique souple, ladite bande étant assemblée sur la poche sur une partie de la périphérie de l'enveloppe de sorte à former un passage entre ladite bande et ladite enveloppe.

Selon un autre mode de réalisation, les moyens d'association temporaire sont formés d'une portion de feuille de matière plastique souple, ladite portion étant associée à la poche sur une partie de la périphérie de l'enveloppe de sorte à former un logement entre ladite portion et ladite enveloppe.

D'autres objets et avantages de l'invention apparaîtront au cours de la description qui suit, en référence aux dessins annexés dans lesquels :
- la figure 1a est une vue de face d'une poche souple comprenant des moyens d'association temporaire du filtre, selon un premier mode de réalisation ;
- la figure 1b représente la poche de la figure 1a sur laquelle un filtre est associé, par l'intermédiaire de moyens d'association temporaire ;
- la figure 2a est une vue de face d'une poche souple comprenant des moyens d'association temporaire du filtre, selon un deuxième mode de réalisation ;
- la figure 2b représente la poche de la figure 2a sur laquelle un filtre est associé, par l'intermédiaire de moyens d'association temporaire ;
- la figure 3 est une vue de face d'une poche souple sur laquelle est associé un filtre par l'intermédiaire de moyens d'association temporaire, selon un troisième mode de réalisation ;
- la figure 4 représente, en vue schématique de face, un système à poches pour la filtration stérile et en circuit clos d'un concentré érythrocytaire,
- la figure 5 représente, en vue schématique de face, un système à poches pour la filtration stérile et en circuit clos du plasma et d'un concentré érythrocytaire.

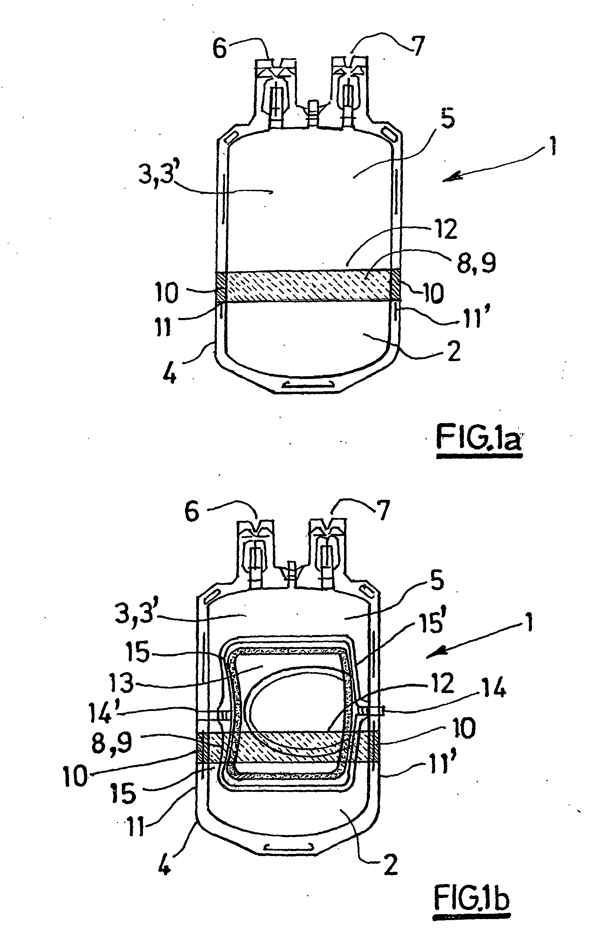

Les figures 1, 2 et 3 représentent une poche souple 1 destinée à recevoir un fluide biologique. Dans un exemple particulier, ce fluide est du sang ou l'un des composants du sang.

Dans les modes de réalisation représentés, la poche 1 est de forme sensiblement rectangulaire. Elle comprend une enveloppe extérieure 2 formée de deux feuilles de plastique souple, soudable et stérilisable 3 et 3', par exemple en PVC. Ces deux feuilles 3 et 3' sont assemblées sur leur périphérie 4, par exemple par soudure, de sorte à définir un volume intérieur 5 pour le fluide.

La poche souple 1 comprend, sur son enveloppe extérieure 2, un orifice d'entrée 6 et un orifice de sortie 7 du fluide. Ces orifices 6 et 7 sont connectés à des tubulures souples, typiquement en matière plastique, destinées à permettre la circulation du fluide dans le système.

Selon un autre mode de réalisation, non représenté, l'entrée et la sortie du fluide peuvent se faire par un seul et même orifice.

La poche souple 1 comprend en outre des moyens d'association temporaire 8, permettant de lui associer au moins un autre élément. Ces moyens d'association temporaire 8 sont disposés sur l'enveloppe extérieure 2. Ainsi, il est possible d'insérer de façon temporaire un élément entre les moyens d'association temporaire 8 et l'enveloppe extérieure 2.

Sur les figures 1a et 1b (premier mode de réalisation), les moyens d'association temporaire 8 sont formés d'une bande de matière plastique souple 9. Cette bande 9 est assemblée, par exemple par soudure, par l'intermédiaire de ses extrémités latérales 10, sur deux bords opposés 11, 11' de la périphérie 4 de la poche 1.

Une ouverture 12 est ménagée entre la bande 9 et l'enveloppe extérieure 2 de la poche 1, de sorte à permettre l'insertion manuelle temporaire de l'élément. La largeur de la bande 9 est prévue pour qu'il soit possible d'insérer l'élément et de le retenir, une fois inséré, contre l'enveloppe extérieure 2 de la poche 1.

Il est ainsi possible de faire passer l'élément entre la bande 9 et l'enveloppe extérieure 2 de la poche 1, de sorte à l'associer temporairement à la poche 1.

Sur la figure 1b, l'élément associé à la poche 1 est un filtre 13. Ce filtre 13, de forme sensiblement rectangulaire, est connecté à la poche 1 par une tubulure 14, et à une autre poche (non représentée) par une tubulure 14'. Les deux tubulures 14 et 14' sont placées sur deux bords opposés 15, 15' du filtre 13, de sorte à faciliter l'insertion du filtre 13 entre la bande 9 et l'enveloppe extérieure 2 de la poche souple 1.

Ce type de filtre souple est par exemple décrit dans le document FR-2 677 883 issu de la demanderesse.

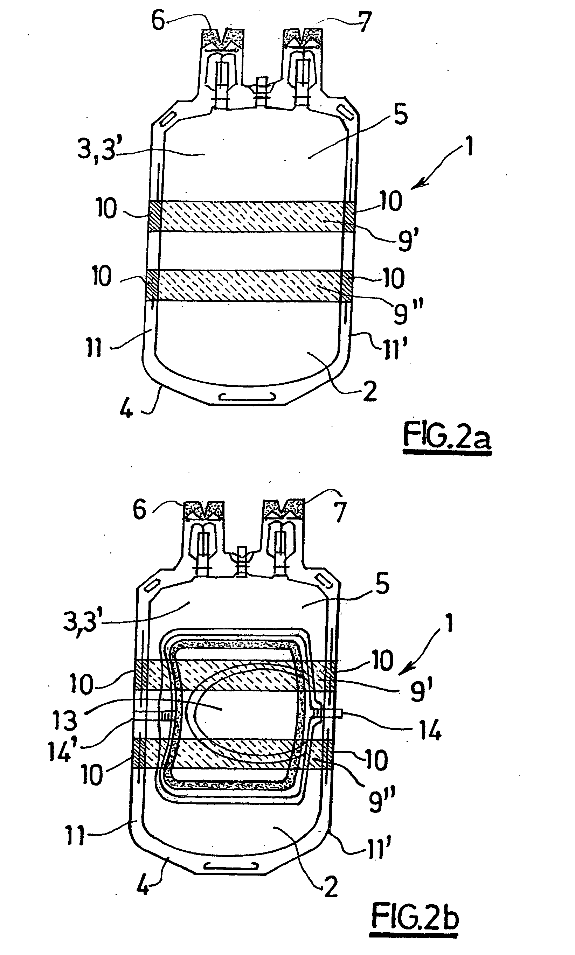

Dans un deuxième mode de réalisation, illustré sur les figures 2a et 2b, les moyens d'association temporaire 8 sont formés de deux bandes de matière plastique souple 9' et 9". Ces deux bandes peuvent être identiques à celle du premier mode de réalisation et sont disposées parallèlement l'une à l'autre, afin d'assurer un meilleur maintien du filtre 13 sur la poche 1.

Dans ce mode de réalisation, les tubulures 14 et 14' peuvent être placées entre les deux bandes 9' et 9", pour améliorer encore ce maintien.

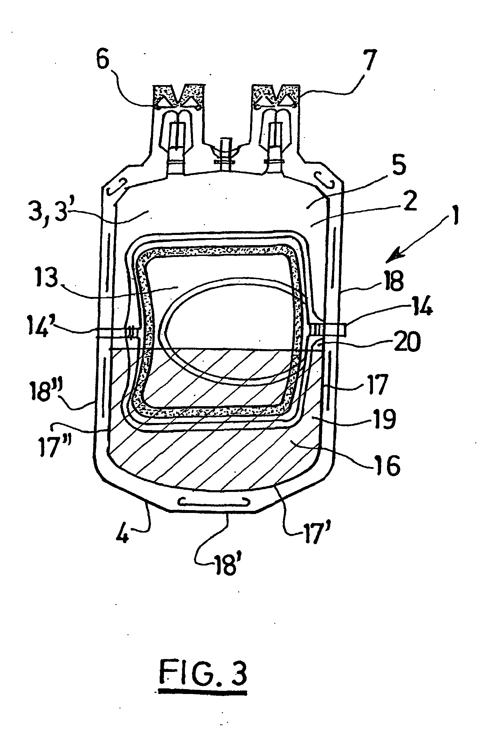

La figure 3 représente un troisième mode de réalisation d'une poche souple 1 comprenant des moyens d'association temporaire 8.

Dans ce mode de réalisation, les moyens d'association temporaire 8 sont formés d'une portion de feuille de matière plastique souple 16. La portion de feuille 16 est de forme sensiblement rectangulaire. Sa surface est environ deux fois moins importante que la surface des feuilles 3 et 3' qui forment l'enveloppe 2.

La portion de feuille 16 est assemblée par l'intermédiaire de trois de ses côtés 17, 17' et 17" à trois bords adjacents 18, 18' et 18" de la périphérie 4 de la poche souple 1.

Ainsi, le filtre 13 peut être inséré temporairement dans un logement 19 formé entre la portion de feuille 16 et l'enveloppe extérieure 2 de la poche 1, par l'intermédiaire d'une ouverture 20.

Dans le mode de réalisation représenté sur la figure 3, le filtre 13 est logé partiellement dans le logement 19, les tubulures 14 et 14' demeurant ainsi à l'extérieur du logement 19.

Dans les modes de réalisation représentés sur les figures 1, 2, et 3, la bande de matière plastique 9, les bandes 9' et 9", et la portion de feuille 16 sont respectivement assemblées à la poche 1 de telle sorte que le filtre 13, une fois inséré, se trouve sensiblement au centre de la poche 1. Cette position centrale facilite la manipulation du système, et permet, lors de l'étape de centrifugation, une meilleure répartition des poids.

En particulier, ce positionnement permet d'éviter au filtre 13 de venir en contact avec le fond du bol, ce qui est source de détérioration du filtre 13.

Les moyens d'association temporaire 8 peuvent être assemblés à la poche 1 par soudage. A cet effet, il est avantageux que les feuilles 3, 3' et respectivement la bande 9, les bandes 9', 9" et la portion de feuille 16 soient réalisées dans le même matériau plastique de sorte à pouvoir, en une seule étape de soudage, réaliser la poche 1 suivant l'invention. En outre, l'assemblage des moyens d'association temporaire 8 sur une partie de la périphérie 4 de l'enveloppe 2 permet une association particulièrement fiable du fait de la résistance et de la stabilité de ladite périphérie.

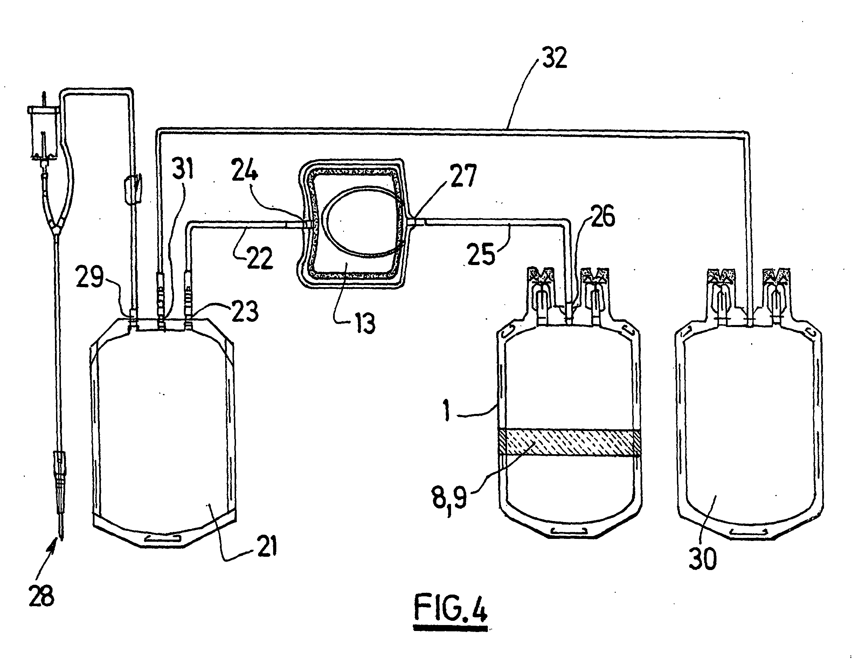

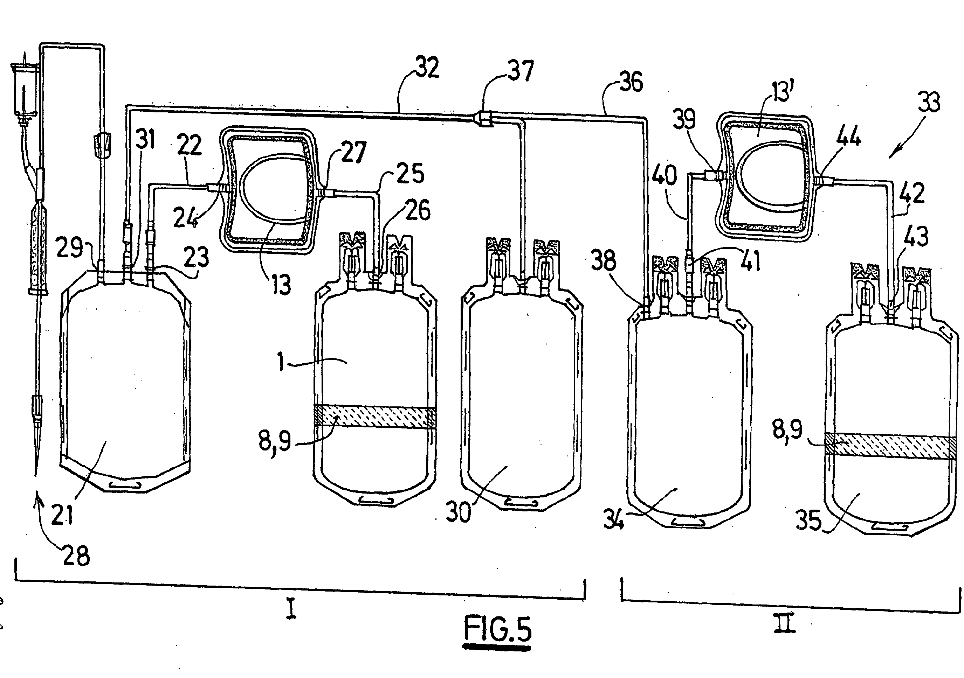

On décrit à présent, en relation avec les figures 4 et 5, un premier et un deuxième mode de réalisation d'un système à poches pour la filtration stérile et en circuit clos d'un fluide biologique, ce système comprenant une poche souple 1 telle que décrite ci-dessus.

Le système à poches comprend une poche primaire 21 destinée à contenir le sang à filtrer, ladite poche primaire 21 étant reliée par l'intermédiaire d'une première tubulure 22, et au niveau de l'un de ses orifices de sortie 23, à l'orifice d'entrée 24 du filtre 13. Une poche 1, dite secondaire, destinée à recevoir le filtrat, est reliée par l'intermédiaire d'une deuxième tubulure 25, et au niveau de l'un de ses orifices d'entrée 26, à l'orifice de sortie 27 du filtre 13.

La poche 1 comprend des moyens d'association temporaire 8 sous la forme d'une bande de matière plastique souple 9, telle que décrite précédemment en relation avec le premier mode de réalisation.

Le système à poches représenté sur la figure 4 comprend en outre des moyens de prélèvement 28 du sang total connectés à un orifice d'entrée 29 de la poche primaire 21, et une poche satellite 30 reliée à un orifice de sortie 31 de la poche primaire 21 par l'intermédiaire d'une troisième tubulure 32.

Dans ce mode de réalisation, le système à poches est typiquement destiné à la filtration stérile et en circuit clos d'un concentré érythrocytaire.

Ce système permet, après avoir été stérilisé, de réaliser en circuit clos les étapes suivantes :
- association du filtre 13 à la poche 1, dite secondaire, par l'intermédiaire d'une bande de matière plastique souple 9 formant moyen d'association temporaire 8 ;
- recueil du sang total dans la poche primaire 21 préalablement remplie d'une solution anticoagulante ;
- centrifugation du système de sorte à séparer le sang en ses composants ;
- dissociation du filtre 13 de la poche 1 ;
- recueil du plasma riche en plaquettes dans la poche satellite 30 ;
- filtration du concentré érythrocytaire et recueil du filtrat dans la poche 1 préalablement remplie d'une solution de conservation.

En variante, l'association du filtre 13 à la poche 1 peut être réalisée postérieurement au recueil du sang total dans la poche primaire 21.

Dans le deuxième mode de réalisation (figure 5), le système à poches est typiquement destiné à la filtration stérile et en circuit clos du plasma et d'un concentré érythrocytaire.

Ce système comprend, dans sa partie I, un système à poches tel que celui décrit en relation avec la figure 4, destiné à la filtration stérile et en circuit clos d'un concentré érythrocytaire, et comprend en outre, une partie II qui est reliée à la partie I par une tubulure 36 connectée à la tubulure 32 par l'intermédiaire d'une jonction 37.

La partie II est formée d'un ensemble 33 comprenant une poche satellite 34, un filtre 13' et une poche satellite 35, l'ensemble 33 étant destiné à la filtration du plasma.

La poche 34, destinée à recueillir le plasma, est reliée, d'une part, au niveau d'un orifice d'entrée 38, à la poche 21 par l'intermédiaire de la tubulure 36, et, d'autre part, au niveau d'un orifice de sortie 41, à un orifice d'entrée 39 du filtre 13' par l'intermédiaire d'une tubulure 40.

La poche 35 est destinée à recevoir le filtrat, c'est-à-dire le plasma déleucocyté. A cet effet, elle est reliée par l'intermédiaire d'une tubulure 42 et au niveau d'un orifice d'entrée 43 à un orifice de sortie 44 du filtre 13'.

Ce système permet, après avoir été stérilisé, de réaliser en circuit clos les étapes suivantes :
- association respectivement des filtres 13 et 13' aux poches 1 et 35, par l'intermédiaire des moyens d'association temporaire 8 ;
- recueil du sang total dans la poche primaire 21 ;
- centrifugation du système de sorte à séparer le sang en ses composants ;
- dissociation du filtre 13 de la poche 1 ;
- recueil du plasma riche en plaquettes dans la poche satellite 30 ;
- filtration du concentré érythrocytaire et recueil du filtrat dans la poche 1 ;
- centrifugation du système de sorte à séparer le plasma riche en plaquettes en une couche de plaquettes et une couche de plasma ;
- dissociation du filtre 13' de la poche 35 ;
- filtration du plasma et recueil du filtrat dans la poche 35.

Selon une réalisation, les tubulures 22, 25, 32, 36, 40 et 42 sont sécables et soudables de sorte à pouvoir, après la filtration, dissocier du système les poches contenant un composant sanguin et permettre ainsi leur éventuelle transfusion ultérieure.

En particulier, préalablement à la deuxième centrifugation, la partie II et la poche 30 sont dissociées du système par coupure et soudure de la tubulure 32.

Dans des modes de réalisation non représentés, la poche 1, 35 peut être associée par l'intermédiaire d'une tubulure à une autre poche pour permettre un traitement ultérieur du filtrat recueilli dans ladite poche 1, 35.

## Revendications

1. Système à poches comprenant au moins une poche souple (1,35) destinée à recevoir un fluide biologique en vue de sa centrifugation, et un filtre (13, 13') connecté à ladite poche (1, 35) par l'intermédiaire d'une tubulure (25, 42), ladite poche souple (1, 35) comprenant une enveloppe extérieure (2) formée de deux feuilles de plastique souple (3, 3') assemblées sur leur périphérie (4) de sorte à définir un volume intérieur (5) pour ledit fluide, ladite enveloppe extérieure (2) étant munie d'au moins un orifice d'entrée (6) et/ou de sortie (7) du fluide, ladite poche (1) comprenant en outre des moyens d'association temporaire (8) du filtre (13, 13') avec ladite poche (1, 35), lesdits moyens (8) étant disposés sur l'enveloppe extérieure (2), ledit système étant **caractérisé en ce que** les moyens d'association temporaire sont assemblés sur la poche (1) sur une partie de la périphérie (4) de l'enveloppe (2) et sont agencés pour permettre l'insertion dudit filtre entre lesdits moyens (8) et ladite enveloppe (2) de sorte à assurer l'association temporaire lors de la centrifugation de la poche (1).

2. Système à poches selon la revendication 1, **caractérisé en ce que** les moyens d'association temporaire (8) sont formés d'au moins une bande de matière plastique souple (9), ladite bande (9) étant assemblée à la poche (1) de sorte à former un passage entre ladite bande (9) et ladite enveloppe (2).

3. Système à poches selon la revendication 2, **caractérisé en ce que** la poche (1, 35) a une forme sensiblement rectangulaire, les extrémités latérales (10) de la bande (9) étant assemblées respectivement sur deux bords opposés (11, 11') de la poche (1, 35).

4. Système à poches selon la revendication 1, **caractérisé en ce que** les moyens d'association temporaire (8) sont formés d'une portion de feuille de matière plastique souple (16), ladite portion (16) étant associée à la poche (1) de sorte à former un logement (19) entre ladite portion (16) et ladite enveloppe (2).

5. Système à poches selon la revendication 4, **caractérisé en ce que** la poche (1) et la portion de feuille (16) ont une forme sensiblement rectangulaire, trois côtés (17, 17', 17") de la portion de feuille (16) étant assemblés respectivement sur trois bords adjacents (18, 18', 18") de la poche (1).

6. Système à poches selon la revendication 5, **caractérisé en ce qu'**il comprend en outre au moins une autre poche (21, 34) connectée au filtre (13, 13') et/ou à la poche (1, 35).

## Claims

1. A system of bags comprising at least one flexible bag (1, 35) intended to receive a biological fluid for the purpose of centrifugation, and a filter (13, 13') connected to the said bag (1, 35) by means of a pipe (25, 42), the said flexible bag (1, 35) comprising an external cover (2) made up of two sheets of flexible plastic (3, 3') joined around their periphery (4) in order to define an interior volume (5) for the said fluid, the said outer cover (2) being provided with at least one fluid input (6) and/or output (7) orifice, the said bag (1) also comprising means (8) for temporary association of the filter (13, 13') with the said bag (1, 35), the said means (8) being arranged on the outer cover (2), the said system being **characterised in that** the temporary association means are joined to the bag (1) on a part of the periphery (4) of the cover (2) and are arranged such as to allow for insertion of the said filter between the said means (8) and the said cover (2) in order to provide the temporary association during the centrifugation of the bag (1).

2. A system of bags according to claim 1, **characterised in that** the temporary association means (8) are made up of at least one band of a flexible plastic material (9), the said band (9) being joined to the bag (1) such as to create a passage between the said band (9) and the said cover (2).

3. A system of bags according to claim 2, **characterised in that** the bag (1, 35) has a substantially rectangular shape, the lateral ends (10) of the band (9) being joined respectively to two opposing edges (11, 11') of the bag (1, 35).

4. A system of bags according to claim 1, **characterised in that** the temporary association means (8) are made up of a portion of a sheet of flexible plastic material (16), the said portion (16) being associated with the bag (1) such as to create a housing (19) between the said portion (16) and the said cover (2).

5. A system of bags according to claim 4, **characterised in that** the bag (1) and the portion of the sheet (16) have a substantially rectangular shape, with three sides (17, 17', 17'') of the portion of the sheet (16) joined respectively to three adjacent edges (18, 18', 18'') of the bag (1).

6. A system of bags according to claim 5, **characterised in that** it also comprises at least one other bag (21, 34) connected to the filter (13, 13') and/or to the bag (1, 35).

## Patentansprüche

1. Beutelsystem mit mindestens einem flexiblen Beutel (1, 35) zur Aufnahme eines biologischen Mediums für sein Zentrifugieren und mit einem über einen Stutzen (25, 42) an den besagten Beutel (1, 35) angeschlossenen Filter (13, 13'), wobei der besagte flexible Beutel (1, 35) einen Außenmantel (2) umfasst, der aus zwei flexiblen Plastikfolien (3, 3') besteht, die derart an ihrem Umfang (4) zusammengesetzt sind, dass sie ein Innenvolumen (5) für das besagte Medium bilden, wobei der besagte Außenmantel (2) mindestens eine Eingangs- und/oder Ausgangsöffnung (6, 7) für das Medium aufweist, wobei der besagte Beutel (1) ferner Mittel (8) für die zeitweilige Verbindung des Filters (13, 13') mit dem besagten Beutel (1, 35) umfasst, wobei die besagten Mittel (8) am Außenmantel (2) angeordnet sind, wobei das besagte System **dadurch gekennzeichnet ist, dass** die Mittel zur zeitweiligen Verbindung an einem Teil des Umfangs (4) des Mantels (2) an dem Beutel (1) zusammengesetzt und so vorgesehen sind, dass sie das Einfügen des besagten Filters zwischen den besagten Mitteln (8) und dem besagten Mantel (2) ermöglichen, um die zeitweilige Verbindung während des Zentrifugierens des Beutels (1) zu gewährleisten.

2. Beutelsystem nach Anspruch 1, **dadurch gekennzeichnet, dass** die Mittel (8) zur zeitweiligen Verbindung aus zumindest einem flexiblen Kunststoffstreifen (9) ausgebildet sind, wobei der besagte Streifen (9) derart mit dem Beutel (1) verbunden ist, dass ein Durchlass zwischen dem besagten Streifen (9) und dem besagten Mantel (2) entsteht.

3. Beutelsystem nach Anspruch 2, **dadurch gekennzeichnet, dass** der Beutel (1, 35) etwa rechteckig ist, wobei die seitlichen Enden (10) des Streifens (9) jeweils mit zwei gegenüberliegenden Rändern (11, 11') des Beutels (1, 35) verbunden sind.

4. Beutelsystem nach Anspruch 1, **dadurch gekennzeichnet, dass** die Mittel (8) zur zeitweiligen Verbindung aus einem Abschnitt einer flexiblen Plastikfolie (16) ausgebildet sind, wobei der besagte Abschnitt (16) derart mit dem Beutel (1) verbunden ist, dass eine Aufnahme (19) zwischen dem besagten Abschnitt (16) und dem besagten Mantel (2) entsteht.

5. Beutelsystem nach Anspruch 4, **dadurch gekennzeichnet, dass** der Beutel (1) und der Folienabschnitt (16) etwa rechteckig sind, wobei drei Seiten (17, 17', 17") des Folienabschnitts (16) jeweils mit drei angrenzenden Rändern (18, 18', 18'') des Beutels (1) verbunden sind.

6. Beutelsystem nach Anspruch 5, **dadurch gekennzeichnet, dass** es ferner mindestens einen weiteren Beutel (21, 34) umfasst, der am Filter (13, 13') und/oder am Beutel (1, 35) angeschlossen ist.
